(19) **Europäisches Patentamt**
**European Patent Office**
**Office européen des brevets**

(11) **EP 2 355 695 B1**

(12) **EUROPEAN PATENT SPECIFICATION**

(45) Date of publication and mention
of the grant of the patent:
**22.06.2016 Bulletin 2016/25**

(51) Int Cl.:
*A61B 5/02* *(2006.01)*      *A61B 5/024* *(2006.01)*
*A61B 5/022* *(2006.01)*      *A61B 5/025* *(2006.01)*

(21) Application number: **09825072.3**

(86) International application number:
**PCT/SG2009/000386**

(22) Date of filing: **20.10.2009**

(87) International publication number:
**WO 2010/053446 (14.05.2010 Gazette 2010/19)**

(54) **METHOD AND SYSTEM FOR MEASURING PARAMETERS OF AUTONOMIC DYSFUNCTION TESTS**

VERFAHREN UND SYSTEM ZUM MESSEN VON PARAMETERN AUTONOMER FEHLFUNKTIONSTESTS

PROCÉDÉ ET SYSTÈME DE MESURE DE PARAMÈTRES LORS DE TESTS DE DYSFONCTION AUTONOME

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **10.11.2008 SG 200808334**

(43) Date of publication of application:
**17.08.2011 Bulletin 2011/33**

(73) Proprietor: **Healthstats International Pte Ltd**
**569139 Singapore (SG)**

(72) Inventors:
• **Ting, Choon Meng**
**Singapore 575584 (SG)**
• **Chua, Ngak Hwee**
**Singapore 539631 (SG)**

(74) Representative: **Bryers LLP**
**7 Gay Street**
**Bath, Bath and North East Somerset BA1 2PH (GB)**

(56) References cited:
**EP-A1- 1 419 730      EP-A1- 1 785 088**
**WO-A1-2006/008535    WO-A1-2006/050725**
**US-A1- 2003 097 075   US-A1- 2003 163 050**
**US-A1- 2006 195 035   US-A1- 2008 045 847**
**US-B1- 6 416 473**

• **ROMANO, S. M. ET AL.: 'Beat-to-Beat Analysis of Pressure Wave Morphology for Pre-Symptomatic Detection of Orthostatic Intolerance During Head-Up Tilt' JOURNAL OF THE AMERICAN COLLEGE OF CARDIOLOGY vol. 44, no. 9, 2004, pages 1891 - 1897, XP004629755**
• **SPRANGERS, R.L.H. ET AL.: 'Initial blood pressure fall on stand up and exercise explained by changes in total peripheral resistance' J APPL PHYSIOL. vol. 70, no. 2, 1991, pages 523 - 530, XP008148707**
• **EWING D J ET AL.: 'Twenty four hour heart rate variability: effects of posture, sleep, and time of day in healthy controls and comparison with bedside tests of autonomic function in diabetic patients' HEART vol. 65, 1991, pages 239 - 244, XP008148712**
• **DUCLA-SOARES J.L. ET AL.: 'Wavelet analysis of autonomic outflow of normal subjects on head-up tilt, cold pressor test, Valsalva manoeuvre and deep breathing' EXP PHYSIOL vol. 92, no. 4, 2007, pages 677 - 686, XP008148708**
• **AKSELROD, S. ET AL.: 'Hemodynamic Regulation: Investigation by Spectral Analysis' AM. J. PHYSIOL. vol. 249, 1985, pages H867 - H875, XP008148711**
• **DEBOER, R.W. ET AL.: 'Beat-to-Beat Variability of Heart Interval and Blood Pressure' AUTOMEDICA vol. 4, 1983, pages 217 - 222, XP008148604**

**(Cont. next page)**

EP 2 355 695 B1

- ZWIENER, U.: 'Physiological Interpretation of Autospectra, Coherence and Phase Spectra of Blood Pressure, Heart Rate, and Respiration Waves in Man' AUTOMEDICA vol. 2, 1978, pages 161 - 169, XP008148605
- DEBOER, R.W. ET AL.: 'Relationship Between Short-term Blood-pressure Fluctuations and Heart-rate variability in Resting Subjects I: A Spectral Analysis Approach' MED. & BIOL. ENG. & COMP. vol. 23, 1985, pages 359 - 364, XP001526219
- AKSELROD, S. ET AL.: 'Power Spectrum Analysis of Heart Rate Fluctuations: A Quantitative Probe of Beat-to-Beat Cardiovascular Control' SCIENCE vol. 213, 1981, pages 220 - 222, XP000617942

**Description**

**FIELD OF THE INVENTION**

The invention relates to a method for measuring parameters of autonomic dysfunction tests and a system for performing same. The method is particularly related to the equipment used.

**BACKGROUND TO THE INVENTION**

The following discussion of the background to the invention is intended to facilitate an understanding of the present invention. However, it should be appreciated that the discussion is not an acknowledgment or admission that any of the material referred to was published, known or part of the common general knowledge in any jurisdiction as at the priority date of the application.

Autonomic dysfunction is presently detected through monitoring the blood pressure and the heart rate changes of a patient through five distinct tests, namely:

- While the patient performs the Valsalva manoeuvre;

- While the patient repeats lying down and standing up (this may be performed by way of a tilt-table);

- While the patient's hand is maintained in a sustained handgrip;

- While the patient undertakes a set exercise regime; and

- While the patient's hand is immersed in cold water.

Existing methods for obtaining blood pressure and heart rate readings during these tests commonly sees the patient being monitored by two or more devices. For example, blood pressure may be measured using a sphygmomanometer or other non-invasive systems, while heart rate is almost universally monitored by way of an electrocardiogram **("ECG").**

These devices are expensive, and, in some cases, may cause additional discomfort to the patient. The use of multiple monitoring devices also may prolong the assessment due to the requirement to calibrate each device separately.

A further problem of the present testing regime for autonomic dysfunction is that there is no integration between the monitoring devices. A side effect of this is that multiple machines, of varying accuracy, have been developed to handle one or more of the tests mentioned above. The results of each such machine then have to be weighted as part of the autonomic dysfunction diagnosis.

An additional side effect to the requirement for multiple machines is that the patient undergoes periods of delay while he or she is disconnected from one monitoring device and hooked up to the next - thereby increasing the overall time necessary to obtain test results.

While the above are problematic in themselves, the use of the ECG during the autonomic dysfunction testing regime introduces further uncertainty into the tests. This is due to the fact that it has been shown that while electrical signals may be sent to the heart, these electrical signals do not always translate into the mechanical motion that is a heart beat. Accordingly, this electrical-mechanical dissociation result in the heart beat as determined by the ECG may not be an accurate reflection of the true heart beat of a patient.

Another drawback to the present testing systems is that the monitoring devices are only capable of qualitatively indicating whether a patient suffers from autonomic dysfunction. There is no way by which the degree or amount of autonomic dysfunction can be quantified using such systems.

It is therefore an object of the present invention to provide a method and system that alleviates, at least in part, one or more of the problems of the prior art mentioned above.

International patent application WO2006050725 A1 relates to a method and device for recording and presentation of physiological parameters, which can include both blood pressure and heart pulse. Information about the heart pulse is derived from a recorded blood pressure signal, whereby the need for an external ECG measurement device is eliminated. It is also describes that a blood pressure measurement is used for the derivation of data such as Heart Rate Variability (HRV) and/or baroreflex sensitivity (BRS) values. United States patent US 6,416,473 B1 describes methods and apparatus for providing a single, accurate indicator of a patient's autonomic nervous system function are described. The indicator is a combination of results of a plurality of different autonomic nervous system tests performed on the patient and referenced to a cross-sectional population.

## SUMMARY OF THE INVENTION

Throughout this document, unless otherwise indicated to the contrary, the terms "comprising", "consisting of", and the like, are to be construed as non-exhaustive, or in other words, as meaning "including, but not limited to".

Additionally, in the context of the remainder of the specification, "beat-to-beat blood pressure" refers to the actual blood pressure assessed on each beat of a patient's heart. It is not a reference to any system which takes an average or uses some other statistical or processing technique to provide a value for each beat of the patient's heart as determined from a period of time longer than one beat of the patient's heart.

The applicant has discovered that the existing understanding that multiple devices are required to assess autonomic dysfunction parameters is in error. This discovery stems from the realisation that a device capable of measuring the beat-to-beat blood pressure of a patient can use the resulting blood pressure waveform data to determine the patient's heart rate. In doing so, as blood pressure is related to the mechanical movement of the heart (ie. heart beats), the potential for error in heart beat calculation latent in ECG systems is avoided. This discovery thus allows for real time measurements of parameters of autonomic dysfunction during performance of the autonomic dysfunction tests through seamless connection with the test system.

In accordance with a first aspect of the invention there is a method for measuring parameters of autonomic dysfunction tests comprising:

obtaining beat-to-beat blood pressure measurements from a patient during the performance of each of at least one autonomic dysfunction test;

calculating variant blood pressure and heart rate values from the beat-to-beat blood pressure measurements for each of the at least one autonomic dysfunction tests;

summing the variant blood pressure values calculated for each autonomic dysfunction test; and

summing the variant heart rate values calculated for each autonomic dysfunction test,

where the variant blood pressure measurement is the absolute value of the blood pressure measurement taken from the patient minus the normal blood pressure measurement stored in a record representing a person having a similar profile as the patient, the absolute value multiplied by a constant; or where the variant heart rate is the absolute value of the heart rate measurement taken from the patient minus the normal heart rate measurement stored in a record representing a person having a similar profile as the patient, the absolute value multiplied by a constant.

The step of calculating variant blood pressure and heart rate values from the beat-to-beat blood pressure measurements preferably includes the sub step of calculating the heart rate according to the formula:

$$hr = 60/d$$

where:

$hr$ is the heart rate of the patient; and

d is the duration of the blood pressure waveform in seconds.

The calculated variant heart rate may be adjusted to factor in the sampling rate of the beat-to-beat blood pressure monitoring device.

The variant blood pressure and heart rate values may be alternatively calculated with reference to a

comparison between the blood pressure measurement taken from the patient against a normal blood pressure measurement stored in a record representing a person without autonomic dysfunction having a similar profile as the patient;

comparison between the calculated heart rate measurement against a normal heart rate measurement stored in a record representing a person without autonomic dysfunction having a similar profile as the patient.

Ideally, the variant blood pressure measurement is the absolute value of the blood pressure measurement taken from the patient minus the normal blood pressure measurement stored in a record representing a person without autonomic dysfunction having a similar profile as the patient, the absolute value multiplied by a constant. Similarly, ideally, the variant heart rate is the absolute value of the heart rate measurement taken from the patient minus the normal heart rate measurement stored in a record representing a person without autonomic dysfunction having a similar profile as the patient, the absolute value multiplied by a constant.

The normal blood pressure measurement is a percentage change in blood pressure of a person without autonomic dysfunction measured during an autonomic dysfunction test preferably determined according to the formula:

$$NBP = (RBP_{ref} - BBP_{ref})/BBP_{ref}$$

where:

NBP is the percentage change in blood pressure of the person without autonomic dysfunction measured during the autonomic dysfunction test; the person of a similar profile as the patient referenced from the external database for the test concerned;

$RBP_{ref}$ is the blood pressure response of the person without autonomic dysfunction; the person of a similar profile as the patient as expected for the autonomic dysfunction test being performed; AND

$BBP_{ref}$ is the resting blood pressure of the person without autonomic dysfunction; the person of a similar profile as the patient as expected for the autonomic dysfunction test being performed.

The actual blood pressure measurement is the actual percentage change in blood pressure of the patient measured during the autonomic dysfunction test, preferably determined according to the formula:

$$ABP = (RBP_{patient} - BBP_{patient})/BBP_{patient}$$

where:

ABP is the actual percentage change in blood pressure of the patient during the autonomic dysfunction test concerned;
$RBP_{patient}$ is the blood pressure response of the patient for the autonomic dysfunction test being performed; AND
$BBP_{patient}$ is the resting blood pressure response of the patient as expected for the autonomic dysfunction test being performed.

The normal heart rate measurement is the percentage change in heart rate of a person without autonomic dysfunction measured during an autonomic dysfunction test, preferably determined according to the formula:

$$NHR = (RHR_{ref} - BHR_{ref})/BHR_{ref}$$

where:

NHR is the percentage change in heart rate of the person without autonomic dysfunction measured during the autonomic dysfunction test; the person of a similar profile as the patient as referenced from the external database for the test concerned;
$RHR_{ref}$ is the heart rate response of the person without autonomic dysfunction; the person of a similar profile as the patient as expected for the autonomic dysfunction test being performed; AND
$BHR_{ref}$ is the resting heart rate of a person without autonomic dysfunction; the person of a similar profile as the patient as expected for the autonomic dysfunction test being performed.

The actual heart rate measurement is the actual percentage change in heart rate of a patient measured during the autonomic dysfunction test determined according to the formula:

$$AHR = (RHR_{patient} - BHR_{patient})/BHR_{patient}$$

where:

AHR is the actual percentage change in heart rate of the patient measured during the autonomic dysfunction test concerned;

$RHR_{patient}$ is the heart rate response of the patient for the autonomic dysfunction test being performed; AND

$BHR_{patient}$ is the resting heart rate of the patient as expected for the autonomic dysfunction test being performed.

The variant blood pressure values and/or the variant heart rate response value may be weighted prior to summation.

The at least one autonomic dysfunction test is at least one of the following:

having the patient perform the Valsalva manoeuvre;

having the patient repeatedly lying down and standing up;

having the patient's hand maintained in a sustained handgrip;

having the patient undertake a set exercise regime; and/or

having the patient's hand immersed in cold water.

Each test is ideally performed for a predetermined length of time.

In accordance with a second aspect of the invention there is a system for measuring parameters of autonomic dysfunction tests comprising:

a beat-to-beat blood pressure monitoring device; and

a processing unit,

where the beat-to-beat blood pressure monitoring device is operable to obtain beat-to-beat blood pressure measurements from a patient during the performance of each of at least one autonomic dysfunction test and calculate variant blood pressure and heart rate values from the beat-to-beat blood pressure measurements for each of the at least one autonomic dysfunction tests, the calculated variant blood pressure and heart rate values being communicated to the processing unit where the variant blood pressure values for each autonomic dysfunction test is summed and the variant heart rate values calculated for each autonomic dysfunction test are summed,
where the variant blood pressure measurement is the absolute value of the blood pressure measurement taken from the patient minus the normal blood pressure measurement stored in a record representing a person having a similar profile as the patient, the absolute value multiplied by a constant; or where the variant heart rate is the absolute value of the heart rate measurement taken from the patient minus the normal heart rate measurement stored in a record representing a person having a similar profile as the patient, the absolute value multiplied by a constant.

In accordance with a third aspect of the invention, being a variant on the second aspect of the invention, there is a system for measuring parameters of autonomic dysfunction tests comprising:

a beat-to-beat blood pressure monitoring device; and

a processing unit,

where the beat-to-beat blood pressure monitoring device is operable to obtain beat-to-beat blood pressure measurements from a patient during the performance of each of at least one autonomic dysfunction test and communicate the measurements to the processing unit, the processing unit thereafter operable to calculate variant blood pressure and heart rate values from the beat-to-beat blood pressure measurements for each of the at least one autonomic dysfunction tests, the processing unit thereafter also operable to sum the variant blood pressure values for each autonomic dysfunction test and sum the variant heart rate values calculated for each autonomic dysfunction test,

where the variant blood pressure measurement is the absolute value of the blood pressure measurement taken from the patient minus the normal blood pressure measurement stored in a record representing a person having a similar profile as the patient, the absolute value multiplied by a constant; or where the variant heart rate is the absolute value of the heart rate measurement taken from the patient minus the normal heart rate measurement stored in a record representing a person having a similar profile as the patient, the absolute value multiplied by a constant.

Preferably, the heart rate is calculated according to the formula:

$$hr = 60/d$$

where:

$hr$ is the heart rate of the patient; and

d is the duration of the blood pressure waveform in seconds.

The calculated heart rate may be adjusted to factor in the sampling rate of the beat-to-beat blood pressure monitoring device.

The blood pressure measurement taken from the patient is, ideally, compared against a normal blood pressure measurement stored in a record representing a person without autonomic dysfunction having a similar profile as the patient in determining the variant blood pressure measurement. Similarly, the calculated heart rate measurement is compared against a normal heart rate measurement stored in a record representing a person without autonomic dysfunction having a similar profile as the patient in determining the variant heart rate measurement.

The variant blood pressure measurement is, preferably, the absolute value of the blood pressure measurement taken from the patient minus the normal blood pressure measurement stored in a record representing a person without autonomic dysfunction having a similar profile as the patient, the absolute value multiplied by a constant. The variant heart rate is the absolute value of the heart rate measurement taken from the patient minus the normal heart rate measurement stored in a record representing a person without autonomic dysfunction having a similar profile as the patient, the absolute value multiplied by a constant.

Preferably, the normal blood pressure measurement is a percentage change in blood pressure of a person without autonomic dysfunction measured during an autonomic dysfunction test determined according to the formula:

$$NBP = (RBP_{ref} - BBP_{ref})/BBP_{ref}$$

where:

NBP is the percentage change in blood pressure of the person without autonomic dysfunction measured during the autonomic dysfunction test; the person of a similar profile as the patient referenced from the external database for the test concerned;

$RBP_{ref}$ is the blood pressure response of the person without autonomic dysfunction; the person of a similar profile as the patient as expected for the autonomic dysfunction test being performed; AND

$BBP_{ref}$ is the resting blood pressure of the person without autonomic dysfunction; the person of a similar profile as the patient as expected for the autonomic dysfunction test being performed.

Preferably, the actual blood pressure measurement is the actual percentage change in blood pressure of the patient measured during the autonomic dysfunction test, determined according to the formula:

$$ABP = (RBP_{patient} - BBP_{patient})/BBP_{patient}$$

where:

ABP is the actual percentage change in blood pressure of the patient during the autonomic dysfunction test concerned;

RBP$_{patient}$ is the blood pressure response of the patient for the autonomic dysfunction test being performed; AND

BBP$_{patient}$ is the resting blood pressure response of the patient as expected for the autonomic dysfunction test being performed.

Preferably, the normal heart rate measurement is the percentage change in heart rate of a person without autonomic dysfunction measured during an autonomic dysfunction test determined according to the formula:

$$NHR = (RHR_{ref} - BHR_{ref})/BHR_{ref}$$

where:

NHR is the percentage change in heart rate of the person without autonomic dysfunction measured during the autonomic dysfunction test; the person of a similar profile as the patient as referenced from the external database for the test concerned;

RHR$_{ref}$ is the heart rate response of the person without autonomic dysfunction; the person of a similar profile as the patient as expected for the autonomic dysfunction test being performed; AND

BHR$_{ref}$ is the resting heart rate of a person without autonomic dysfunction; the person of a similar profile as the patient as expected for the autonomic dysfunction test being performed.

Preferably, the actual heart rate measurement is a percentage change in heart rate of the patient measured during the autonomic dysfunction test as determined according to the formula:

$$AHR = (RHR_{patient} - BHR_{patient})/BHR_{patient}$$

where:

AHR is the actual percentage change in heart rate of the patient measured during the autonomic dysfunction test concerned;

RHR$_{patient}$ is the heart rate response of the patient for the autonomic dysfunction test being performed; AND

BHR$_{patient}$ is the resting heart rate of the patient as expected for the autonomic dysfunction test being performed.

The variant blood pressure values determined for each autonomic dysfunction test may be weighted before summation. Similarly, the variant heart rate values determined for each autonomic dysfunction test may be weighted before summation.

The at least one autonomic dysfunction test is at least one of the following:

having the patient perform the Valsalva manoeuvre;
having the patient repeatedly lying down and standing up;
having the patient's hand maintained in a sustained handgrip;
having the patient undertake a set exercise regime; and/or
having the patient's hand immersed in cold water.

Each of the at least one autonomic dysfunction test is performed for a predetermined length of time.

Preferably, the beat-to-beat blood pressure monitoring device is a non-invasive blood pressure monitoring service.

Communication of measurements between the beat-to-beat blood pressure monitoring device and the processing unit may be by way of wired or wireless means.

The record of the person without autonomic dysfunction having a similar profile as the patient preferably include information in respect of at least one of the following attributes: age; sex; about the same health profile; ethnicity, and about the same medication regime.

In accordance with a fourth aspect of the invention there is a computer readable medium having software stored thereon that, when executed by an appropriate processor, performs a method for measuring parameters of autonomic dysfunction tests according to the first aspect of the invention.

**BRIEF DESCRIPTION OF THE DRAWINGS**

The invention will now be described, by way of example only, with reference to the accompanying drawings, in which:

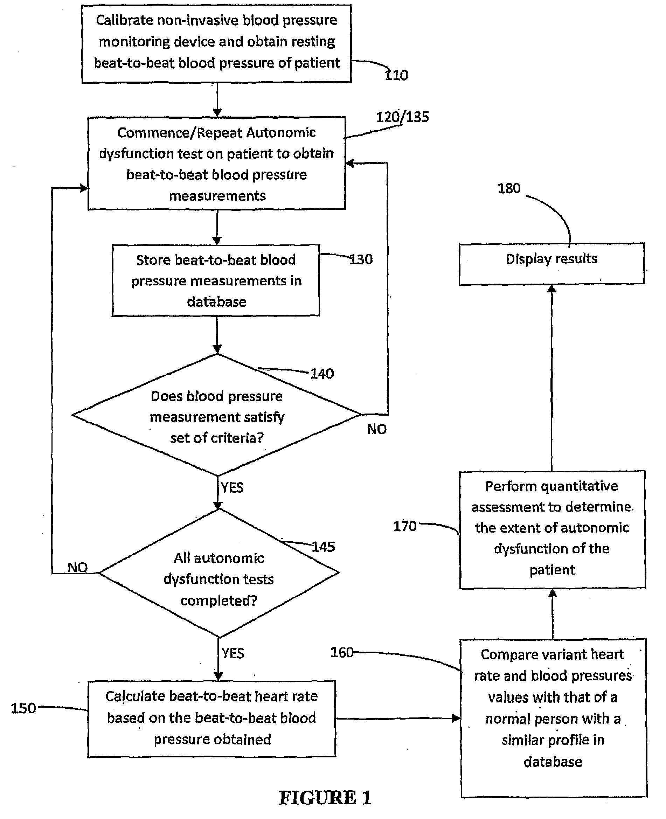

Figure 1 is a flow chart of the method for the measurement of parameters of autonomic dysfunction tests in accordance to the first embodiment of the invention.

Figure 2 is an illustration of a series of beat-to-beat blood pressure waveform cycles.

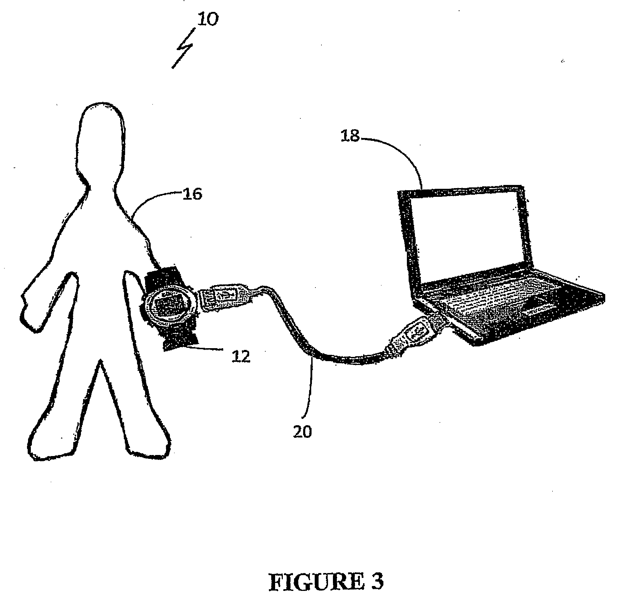

Figure 3 is a system setup for the measurement of parameters of autonomic dysfunction tests.

**PREFERRED EMBODIMENTS OF THE INVENTION**

In accordance with a first embodiment of the invention there is a system **10** for measuring parameters of autonomic dysfunction tests of a patient **16** comprising:

- a non-invasive, beat-to-beat blood pressure monitoring device **12;** and
- an autonomic dysfunction analysis device

The non-invasive, beat-to-beat blood pressure monitoring device **12** is ideally the BPro™ device available from HealthSTATS International Pte Ltd of Singapore. The BPro™ device measures treal time beat-to-beat arterial pulse wave and is able to determine the beat-to-beat blood pressure accurately.

The autonomic dysfunction analysis device, in this embodiment, takes the form of a notebook computer **18.** The notebook computer **18** is in data communication with the non-invasive, beat-to-beat blood pressure monitoring device **12** by way of a Universal Serial Bus **("USB")** cable **20.**

A hard drive of the notebook computer **18** has software recorded thereon such that, when the software is executed, it obtains data from the non-invasive, beat-to-beat blood pressure monitoring device **12** and is able to analyse the autonomic dysfunction parameters as required. The software also has reference to a database. In this embodiment, the database is an external database. Each record in the external database includes the expected blood pressure and heart rate profiles for a "normal" patient segregated according to age and sex. In determining whether the patient **16** has autonomic dysfunction, comparison will be made with a 'normal' person having a similar profile, where they are about the same age and sex.

The system **10** will now be described in more detail in the context of its intended operation.

A medical personnel such as a doctor or a nurse, attaches the non-invasive blood pressure monitoring device **12** to the patient **16** at a location where the patient's **16** blood pressure can be measured. The non-invasive blood pressure monitoring device **12** is also connected to the notebook computer **18**. The medical personnel then executes the software stored on the hard drive (if not already operating) to control the testing regime to follow.

Prior to commencement of the autonomic dysfunction tests, the non-invasive blood pressure monitoring device **12** is calibrated to capture bio-data about the patient **16.** As such a calibration would be known to the person skilled in the art, it will not be described in more detail here. In addition to calibration, the resting beat-to-beat blood pressure measurement of the patient **16** is obtained (Step **110).** Beat-to-beat blood pressure measurements are taken by the non-invasive blood pressure monitoring device **12** from at least this point forward.

The patient **16** is then subjected, to each test one after the other in the set of autonomic dysfunction tests of the prior art while wearing the non-invasive blood pressure monitoring device **12** (Step **120).** For the sake of clarity, these tests are:

- having the patient perform the Valsalva manoeuvre;

- having the patient repeatedly lying down and standing up;

- having the patient's hand maintained in a sustained handgrip;

- having the patient undertake a set exercise regime; and

- having the patient's hand immersed in cold water.

All throughout this testing regime, beat-to-beat blood pressure data is being accumulated by the non-invasive blood pressure monitoring device **12** and relayed back to the notebook computer **18** by way of the USB cable **20.** This information is then stored in a blood pressure database and also recorded on the hard drive of the notebook computer **18** (Step **130).**

As part of each test, the software executing on the notebook computer **18** indicates to the medical personnel **22** the test to be performed prior to commencement. Similarly, the software indicates the exact time that the test is to commence as well as the exact time that the test is to finish. In this manner, each test is performed for a predetermined length of time - typically between twenty second and five minutes as required by the standard protocols of the country in which the test is being performed.

The expected consequences of each test, and how they relate to whether a patient may suffer from autonomic dysfunction, are as follows:

**The Valsalva Manoeuvre-** The normal physiological response consists of four phases: an initial pressure rise phase, a reduced venous return and compensation phase, a pressure release phase and a return of cardiac output phase. If there is little or no variation in heart rate throughout the four phases of the test, the test is indicative that the patient suffers from autonomic dysfunction.

**Repeated Transience Between Standing Position and Lying Position-** If there is a difference in the systolic blood pressure of the patient between standing and lying positions of greater than 30mmHg and a resultant increase in heart rate, the test is indicative that the patient suffers from autonomic dysfunction.

**Sustained Handgrip-**If a related increase in heart rate and systolic blood pressure is evident, the test is <u>not</u> indicative that the patient suffers from autonomic dysfunction.

**Exercise Regime.** If the heart rate and blood pressure do not elevate to a sustained plateau pattern, the test is indicative that the patient suffers from autonomic dysfunction.

**Placement of Hand in Cold Water-** If the test shows that the patient does not show a sudden and sharp increase in both heart rate and blood pressure which settles over time (heart rate settling first), the test is indicative that the patient suffers from autonomic dysfunction.

At the end of each test, the notebook computer **18** reviews the results obtained against a set of criteria aimed at determining whether the test has been conducted properly and are of sufficient quality for analysis (Step **140).** Such criteria include identifying any stray values. If the results do not meet these criteria the software indicates to the medical personnel **22** that the test should be repeated (Step **135).** Other criteria aimed at determining whether the test has been conducted properly and is of sufficient quality for analysis can not be assessed by the notebook computer **18.** Accordingly, mechanisms are also provided to allow the medical personnel **22** to manually indicate that a test should be repeated.

If the results meet the criteria, the software operates to direct the medical personnel to perform the next test in the testing regime. Of course, if the last test meets the criteria, the software operates to perform the analysis as described (Step **145).**

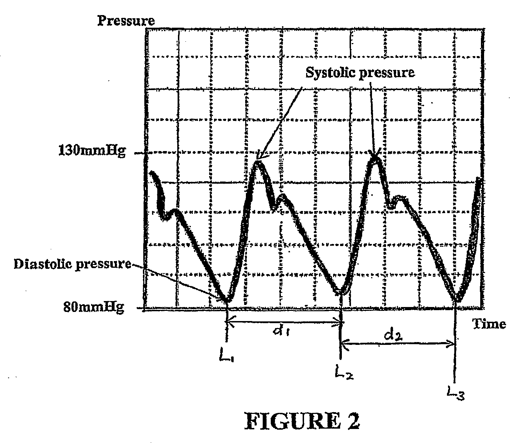

Once all of the information has been obtained from the non-invasive blood pressure monitoring device **12,** the software operates to determine the duration of each blood pressure waveform (Step **150).** In this embodiment, this is achieved by analysing the data as a whole and determining the lowest point (L) in each waveform cycle as illustrated graphically in Figure 2.

With the duration of each blood pressure waveform determined, it is possible to determine the heart rate of the patient at the time the waveform was obtained by the equation:

$$hr = 60/d$$

where *hr* is the heart rate and d is the duration of the blood pressure waveform in seconds.

This then means that a blood pressure reading and a heart rate measurement is available at any time during testing.

With the blood pressure and heart rate values during each test able to be referenced, the software compares the heart rate and blood pressure profiles to those stored in the external database for a person falling into a similar age range as the patient who is also of a similar sex (Step **160).** From this comparison, a numerical assessment of autonomic dysfunction for the test is calculated according to the following formulas:

$$hrad_{test} = |NHR - AHR| \times 100\%;$$

and

$$bpad_{test} = |NBP - ABP| \times 100\%$$

where:

NHR is the percentage change in heart rate of a person without autonomic dysfunction of a similar sex and age as referenced from the external database for the test concerned; the formula for calculating the NHR value is set out in more detail below.

AHR is the actual percentage change in heart rate of the patient during the test concerned. The formula for calculating the AHR value is set out in more detail below.

NBP is the percentage change in blood pressure of a person without autonomic dysfunction of a similar sex and age as referenced form the external database for the test concerned. The formula for calculating the NBP value is set out in more detail below; and

ABP is the actual percentage change in blood pressure of the patient during the test concerned. The formula for calculating the ABP value is set out in more detail below.

In each case the absolute difference between the two values (EHR and AHR; EBP and ABP) and the extent of the variation is of primary importance. The direction of the variation is considered essentially immaterial.

As mentioned above, the NHR, AHR, NBP and ABP are calculated values. The formula for each value is as follows:

$$NHR = (RHR_{ref} - BHR_{ref})/BHR_{ref}$$

$$AHR = (RHR_{patient} - BHR_{patient})/BHR_{patient}$$

$$NBP = (RBP_{ref} - BBP_{ref})/BBP_{ref}$$

$$ABP = (RBP_{patient} - BBP_{patient})/BBP_{patient}$$

Where

$RHR_{ref}$ is the heart rate response of a person of the same sex and age as referenced from the external database for the test concerned. The measurement for $RHR_{ref}$ varies for different autonomic dysfunction tests conducted. For example, $RHR_{ref}$ for the Exercise Regime test and sustained handgrip test is taken to be the maximum heart rate measured during the conduct of the tests. For the Valsalva Manoeuvre test, $RHR_{ref}$ is taken to be the sum of the minimum, maximum and/or the 1st derivate at different phases of the test. The expected $RHR_{ref}$ for the remaining tests would be known to the person skilled in the art.

$BHR_{ref}$ is the resting heart rate of a person of a similar sex and age as referenced from the external database for the test concerned.

$RHR_{patient}$ is the heart rate response of the patient for a similar description above.

$BHR_{patient}$ is the resting heart rate of the patient.

$RBP_{ref}$ is the blood pressure response of a person of a similar sex and age as referenced from the external database for the test concerned. The measurement for $RBP_{ref}$ varies for different autonomic dysfunction tests conducted and is known to a person skilled in the art. For example, $RBP_{ref}$ for the Exercise Regime test and sustained handgrip test is taken to be the maximum systolic blood pressure values measured during the conduct of the tests. Again, the expected $RBP_{ref}$ value for the remaining tests would be known to the person skilled in the art.

$BBP_{ref}$ is the resting blood pressure of a person of a similar sex and age as referenced from the external database for the test concerned.

$RBPp_{atient}$ is the blood pressure response of the patient for a similar description above.

$BBP_{patient}$ is the resting blood pressure of the patient.

The final quantitative assessment of autonomic dysfunction parameters is then expressed as the sum total of the $hrad_{test}$ and $bpad_{test}$ values for each test (Step **170).** This value then may be graphed to show how the patient's trend of autonomic function and if there is any worsening/improvement in his condition (Step **180).**

It should be appreciated by the person skilled in the art that the above invention is not limited to the embodiment described. In particular, the following modifications and improvements may be made without departing from the scope of the present invention:

- The beat-to-beat blood pressure monitoring device **12** need not be non-invasive. While a non-invasive system is preferable for the comfort of the patient, the invention as described could just as easily be adapted for use with an invasive beat-to-beat blood pressure monitoring device **12.**
- The beat-to-beat blood pressure monitoring device **12** may have separate processing circuits or software that allows for calculation of the heart rate as described above. In this situation, the beat-to-beat blood pressure monitoring device **12** may provide the heart rate and beat-to-beat blood pressure data to the autonomic dysfunction analysis device **14.** This then alleviates the autonomic dysfunction analysis device **14** of the need to calculate the heart rate itself.
- The beat-to-beat blood pressure monitoring device **12** and autonomic dysfunction analysis device **14** may be integrated into a single device.
- The software may allow for the medical personnel **22** to override the assessment of the test measurements against the set criteria. In this manner, the medical personnel can force the system to repeat any test he or she deems necessary to repeat.
- In situations where the beat-to-beat blood pressure monitoring device **12** physically connects to the autonomic dysfunction analysis device **14,** the method of connection can be by means other than USB cable **20.** For instance, connection can be achieved by way of a Fire wire cable or serial cable.
- Wireless connection between the beat-to-beat blood pressure monitoring device **12** and the autonomic dysfunction analysis device **14** may be by way of infrared or wireless radio.
- The autonomic dysfunction analysis device **14** need not be a notebook computer **18.** It can just as easily be a dedicated device or desktop computer, or a terminal attached to a computer network.
- While it is preferable that an analysis is performed during all the full set of tests, it is possible for a similar to be based on the results of one or more of the tests rather than the full test set.
- A start/stop button may be provided on the beat-to-beat blood pressure monitoring device **12** as a means of indicating the commencement and finishing of each test as directed by the medical personnel **22.**
- The non-invasive beat-to-beat blood pressure monitoring device **12** need not be connected to the notebook computer **18** during testing. Instead, the non-invasive beat-to-beat blood pressure monitoring device **12** may have storage means for storing blood pressure measurements. These measurements can then be transferred form the storage means to the notebook computer **18** upon subsequent linkup following completion of the last test.
- In yet a further alternative arrangement, the system may continuously monitor the blood pressure of the patient during the testing regime. In this arrangement, measurements relating to particular tests may be determined by filtering out those measurements considered indicative of the rest heartbeat of the patient.
- In addition to the heart rate and blood pressure response to each test, the settling time or response time may be used to provide an indication on whether a patient is suffering from autonomic dysfunction
- Ideally, the non-invasive blood pressure monitoring device is positioned adjacent to an artery.
- The software may be implemented as an electronic circuit or may be externally stored relative to the autonomic dysfunction analysis device **14.**
- As the sampling rate of the beat-to-beat blood pressure monitoring device **12** may impact on the calculated heart rate, the formula used to determine the calculated heart rate may be amended to factor in the influence of such a sampling rate.
- The calculated values hrad and bpad may be further corrected to account for co-existing conditions that may influence such values. An example is to correct the values to account for the patient taking beta-blockers.

- Each record held by the external database may be further segregated according to ethnicity with the NHR and NBP values being correlated on the basis of ethnicity in addition to age and sex. Even further segregation of records held in the external database can be made with reference to any other relevant health profile as would be known to the person skilled in the art.
- The $RBP_{ref}$, $RHR_{ref}$, $BBP_{ref}$ and $BHR_{ref}$ values may be determined by averaging the relevant blood pressure measurements and heart rates of a 'normal' person found in the external database.
- The blood pressure response and heart rate response to an autonomic dysfunction test for a 'normal' person may be a range of values bounded by upper and lower limits.
- The hrad and bpad values may be weighted according to the significance of the test providing such values. In this respect, it is noted that higher weightings could be applied to the Valsalva manoeuvre and cold water tests due to the observation that indications of autonomic dysfunction only show up in these tests when the disease is more pronounced.

It should be further appreciated by the person skilled in the art that the above modifications and improvements, where not mutually exclusive, can be combined to form yet further embodiments considered within the scope of the present invention.

**Claims**

1. A system for measuring parameters of autonomic dysfunction tests wherein the system comprises:

> a beat-to-beat blood pressure monitoring device (12); and
> a processing unit (18),
> and **characterised in that** the beat-to-beat blood pressure monitoring device is operable to either:

> obtain beat-to-beat blood pressure measurements from a patient (16) during the performance of each of at least one autonomic dysfunction test and calculate variant blood pressure, heart rate and variant heart rate values from the beat-to-beat blood pressure measurements for each of the at least one autonomic dysfunction tests, the calculated variant blood pressure and variant heart rate values being communicated to the processing unit where the variant blood pressure values for each autonomic dysfunction test are summed and the variant heart rate values calculated for each autonomic dysfunction test are summed; or the beat-to-beat blood pressure monitoring device (12) is operable to obtain beat-to-beat blood pressure measurements from a patient during the performance of each of at least one autonomic dysfunction test and communicate the measurements to the processing unit (18), the processing unit thereafter operable to calculate variant blood pressure, heart rate and variant heart rate values from the beat-to-beat blood pressure measurements for each of the at least one autonomic dysfunction tests, the processing unit (18) thereafter also operable to sum the variant blood pressure values for each autonomic dysfunction test and sum the variant heart rate values calculated for each autonomic dysfunction test, where the variant blood pressure measurement is the absolute value of the blood pressure measurement taken from the patient minus the normal blood pressure measurement stored in a record representing a person having a similar profile as the patient, the absolute value multiplied by a constant; or where the variant heart rate is the absolute value of the heart rate measurement taken from the patient minus the normal heart rate measurement stored in a record representing a person having a similar profile as the patient, the absolute value multiplied by a constant.

2. A method for measuring parameters of autonomic dysfunction tests, the method comprising obtaining beat-to-beat blood pressure measurements from a patient during the performance of each of at least one autonomic dysfunction test; and **characterised in that** the method further comprises the steps of: calculating heart rate, variant blood pressure and variant heart rate values from the beat-to-beat blood pressure measurements for each of the at least one autonomic dysfunction tests; summing the variant blood pressure values calculated for each autonomic dysfunction test; and summing the variant heart rate values calculated for each autonomic dysfunction test, where the variant blood pressure measurement is the absolute value of the blood pressure measurement taken from the patient minus the normal blood pressure measurement stored in a record representing a person having a similar profile as the patient, the absolute value multiplied by a constant; or where the variant heart rate is the absolute value of the heart rate measurement taken from the patient minus the normal heart rate measurement stored in a record representing a person having a similar profile as the patient, the absolute value multiplied by a constant.

3.  A system or method according to claim 1 or 2, where the step of calculating variant blood pressure and heart rate values from the beat-to-beat blood pressure measurements includes the sub step of calculating the heart rate according to the formula:

$$hr = 60/d$$

where:

>   $hr$ is the heart rate of the patient; and
>   $d$ is the duration of the blood pressure waveform in seconds.

4.  A system or method according to claim 3, where the calculated heart rate or the sub step of calculating the heart rate is adjusted to factor in the sampling rate of the beat-to-beat blood pressure monitoring device (12).

5.  A system or method according to any one of claims 1 to 4, wherein for a system, the blood pressure measurement taken from the patient is compared against a normal blood pressure measurement stored in a record representing a person having a similar profile as the patient in determining the variant blood pressure measurement; the calculated heart rate measurement is compared against a normal heart rate measurement stored in a record representing a person having a similar profile as the patient in determining the variant heart rate measurement; and wherein for a method, the step of calculating variant blood pressure and heart rate values from the beat-to-beat blood pressure measurements includes the sub steps of:

>   comparing the blood pressure measurement taken from the patient against a normal blood pressure measurement stored in a record representing a person without autonomic dysfunction having a similar profile as the patient;
>   comparing the calculated heart rate measurement against a normal heart rate measurement stored in a record representing a person without autonomic dysfunction having a similar profile as the patient.

6.  A system or method according to any preceding claim, where the normal blood pressure measurement is the percentage change in blood pressure of a person without autonomic dysfunction measured during an autonomic dysfunction test, determined according to the formula:

$$NBP = (RBP_{ref} - BBP_{ref})/BBP_{ref}$$

where:

>   NBP is the percentage change in blood pressure of the person without autonomic dysfunction measured during the autonomic dysfunction test; the person of a similar profile as the patient referenced from the external database for the test concerned;
>   $RBP_{ref}$ is the blood pressure response of the person without autonomic dysfunction; the person of a similar profile as the patient as expected for the autonomic dysfunction test being performed; AND
>   $BBP_{ref}$ is the resting blood pressure of the person without autonomic dysfunction; the person of a similar profile as the patient as expected for the autonomic dysfunction test being performed.

7.  A system or method according to claim 6, where the actual blood pressure measurement is the actual percentage change in blood pressure of the patient measured during the autonomic dysfunction test determined according to the formula:

$$ABP = (RBP_{patient} - BBP_{patient})/BBP_{patient}$$

where:

ABP is the actual percentage change in blood pressure of the patient during the autonomic dysfunction test concerned;
$RBP_{patient}$ is the blood pressure response of the patient for the autonomic dysfunction test being performed; AND
$BBP_{patient}$ is the resting blood pressure response of the patient as expected for the autonomic dysfunction test being performed.

8.  A system or method according to any preceding claim, where the normal heart rate measurement is the percentage change in heart rate of a person without autonomic dysfunction measured during an autonomic dysfunction test, determined according to the formula:

$$NHR = (RHR_{ref} - BHR_{ref})/BHR_{ref}$$

where:

NHR is the percentage change in heart rate of the person without autonomic dysfunction measured during the autonomic dysfunction test; the person of a similar profile as the patient as referenced from the external database for the test concerned;
$RHR_{ref}$ is the heart rate response of the person without autonomic dysfunction; the person of a similar profile as the patient as expected for the autonomic dysfunction test being performed; AND
$BHR_{ref}$ is the resting heart rate of a person without autonomic dysfunction; the person of a similar profile as the patient as expected for the autonomic dysfunction test being performed.

9.  A system or method according to claim 1 or claim 8, where the actual heart rate measurement is the actual percentage change in heart rate of the patient measured during the autonomic dysfunction test, determined according to the formula:

$$AHR = (RHR_{patient} - BHR_{patient})/BHR_{patient}$$

where:

AHR is the actual percentage change in heart rate of the patient measured during the autonomic dysfunction test concerned;
$RHR_{patient}$ is the heart rate response of the patient for the autonomic dysfunction test being performed; AND
$BHR_{patient}$ is the resting heart rate of the patient as expected for the autonomic dysfunction test being performed.

10. A system or method according to any one of the preceding claims, where the variant blood pressure values determined for each autonomic dysfunction test are weighted before summation; and/or the variant heart rate values determined for each autonomic dysfunction test are weighted before summation.

11. A system or method according to any one of the preceding claims, where the at least one autonomic dysfunction test is at least one of the following:

having the patient perform the Valsalva manoeuvre;
having the patient repeatedly lying down and standing up;
having the patient's hand maintained in a sustained handgrip;
having the patient undertake a set exercise regime; and/or
having the patient's hand immersed in cold water.

12. A system or method according to any one of the preceding claims, where each of the at least one autonomic dysfunction test is performed for a predetermined length of time.

13. A system or method according to any one of the preceding claims, where the beat-to-beat blood pressure monitoring device (12) is a non-invasive blood pressure monitoring service.

14. A system according to claim 1, where the beat-to-beat blood pressure monitoring device (12) is configured to obtain real time beat-to-beat arterial pulse wave.

15. A system according to claim 1, where communication of measurements between the beat-to-beat blood pressure monitoring device (12) and the processing unit (18) is by way of wireless means.

16. A system according to any one of claim 1, where the record of the person having a similar profile as the patient includes information in respect of at least one of the following attributes: age; sex; health profile; ethnicity.

17. A computer readable medium having software stored thereon that, when executed by a processor of a system according to claim 1, performs a method for measuring parameters of autonomic dysfunction tests according to any one of claims 2 to 13.

**Patentansprüche**

1. System zum Messen von Parametern von autonomen Fehlfunktionstests, wobei die Anlage umfasst:

eine pulsweise (Schlag-auf-Schlag-) Blutdruck-Überwachungseinrichtung (12) und
eine Verarbeitungseinheit (18),
**dadurch gekennzeichnet, dass** die pulsweise Blutdruck-Überwachungseinrichtung betreibbar ist,

- um entweder pulsweise Blutdruckmesswerte von einem Patienten (16) während des Ausführens jedes von zumindest einem autonomen Fehlfunktionstest zu erhalten und abweichende Blutdruck-, Herzfrequenz- und abweichende Herzfrequenzwerte aus den pulsweisen Blutdruckmesswerten für jeden des zumindest einen autonomen Fehlfunktionstests zu berechnen, wobei die berechneten abweichenden Blutdruck- und abweichenden Herzfrequenzwerte zu der Verarbeitungseinheit kommuniziert werden, wo die abweichenden Blutdruckwerte für jeden autonomen Fehlfunktionstest summiert werden und wo die für jeden autonomen Fehlfunktionstest berechneten abweichenden Herzfrequenzwerte summiert werden,

oder

- die pulsweise Blutdruck-Überwachungseimichtung (12) ist betreibbar, um pulsweise Blutdruckmesswerte von einem Patienten während des Ausführens jedes von zumindest einem autonomen Fehlfunktionstest zu erzielen und die Messwerte zu der Verarbeitungseinheit (18) zu kommunizieren, die danach betreibbar ist, um abweichende Blutdruck-, Herzfrequenz- und abweichende Herzfrequenzwerte aus den pulsweisen Blutdruckmesswerten für jeden des zumindest einen autonomen Fehlfunktionstests zu berechnen, wobei die Verarbeitungseinheit (18) danach auch betreibbar ist, um die abweichenden Blutdruckwerte für jeden autonomen Fehlfunktionstest zu summieren und um die für jeden autonomen Fehlfunktionstest berechneten abweichenden Herzfrequenzwerte zu summieren,
- wobei der abweichende Blutdruckmesswert der absolute Wert des von dem Patienten gewonnenen Blutdruckmesswerts abzüglich des in einer Aufzeichnung gespeicherten normalen Blutdruckmesswerts ist, welcher kennzeichnend ist für eine Person, die ein ähnliches Profil aufweist wie der Patient, wobei der absolute Wert mit einer Konstanten multipliziert wird,
- oder wobei die abweichende Herzfrequenz der absolute Wert des von dem Patienten gewonnenen Herzfrequenzmesswerts abzüglich des in einer Aufzeichnung gespeicherten normalen Herzfrequenzmesswerts ist, welcher kennzeichnend ist für eine Person, die ein ähnliches Profil aufweist wie der Patient, wobei der absolute Wert mit einer Konstanten multipliziert wird.

2. Verfahren zum Messen von Parametern von autonomen Fehlfunktionstests, wobei das Verfahren umfasst:

Erfassen von pulsweisen (Schlag-auf-Schlag-) Blutdruckmesswerten von einem Patienten während des Ausführens jedes von zumindest einem autonomen Fehlfunktionstest,
**dadurch gekennzeichnet, dass** das Verfahren ferner die Schritte umfasst:

- Berechnen von Herzfrequenz-, abweichenden Blutdruck- und abweichenden Herzfrequenzwerten aus den pulsweisen Blutdruckmesswerten für jeden des zumindest einen autonomen Fehlfunktionstests,
- Summieren der für jeden autonomen Fehlfunktionstest berechnenden abweichenden Blutdruckwerte und

- Summieren der für jeden autonomen Fehlfunktionstest berechneten abweichenden Herzfrequenzwerte,
- wobei der abweichende Blutdruckmesswert der absolute Wert des von dem Patienten gewonnenen Blutdruckmesswerts abzüglich des in einer Aufzeichnung gespeicherten Blutdruckmesswerts ist, welcher kennzeichnend ist für eine Person, die ein ähnliches Profil aufweist wie der Patient, wobei der absolute Wert mit einer Konstanten multipliziert wird,
- oder wobei die abweichende Herzfrequenz der absolute Wert des von dem Patienten gewonnenen Herzfrequenzmesswerts abzüglich des in einer Aufzeichnung gespeicherten normalen Herzfrequenzmesswerts ist, welcher kennzeichnend ist für eine Person, die ein ähnliches Profil aufweist wie der Patient, wobei der absolute Wert mit einer Konstanten multipliziert wird.

3.  System oder Verfahren nach Anspruch 1 oder 2, wobei der Schritt des Berechnens der abweichenden Blutdruck- und Herzfrequenzwerte aus den pulsweisen Blutdruckmesswerten den Unterschritt des Berechnens der Herzfrequenz entsprechend der Formel enthält:

$$hr = 60/d,$$

wobei

$hr$ die Herzfrequenz des Patienten ist und
d die Dauer des Blutdruck-Kurvenverlaufs in Sekunden ist.

4.  System oder Verfahren nach Anspruch 3, wobei die berechnete Herzfrequenz oder der Unterschritt des Berechnens der Herzfrequenz auf den Faktor in der Abtastrate der pulsweisen Blutdruck-Überwachungseinrichtung (12) eingestellt wird.

5.  System oder Verfahren nach einem der Ansprüche 1 bis 4,

- wobei bei einer Anlage der von dem Patienten gewonnene Blutdruckmesswert gegenüber einem in einer Aufzeichnung gespeicherten normalen Blutdruckmesswelt, welcher kennzeichnend ist für eine Person, die ein ähnliches Profil aufweist wie der Patient, bei der Bestimmung des abweichenden Blutdruckmesswerts verglichen wird,
- wobei der berechnete Herzfrequenzmesswert gegenüber einem in einer Aufzeichnung gespeicherten normalen Herzfrequenzmesswert, welcher kennzeichnend ist für eine Person, die ein ähnliches Profil aufweist wie der Patient, bei der Bestimmung des abweichenden Herzfrequenzmesswerts verglichen wird,
- und wobei bei einem Verfahren der Schritt des Berechnens der abweichenden Blutdruck- und Herzfrequenzwerte aus den pulsweisen Blutdruckmesswerten die Unterschritte enthält:
- Vergleichen des von dem Patienten gewonnenen Blutdruckmesswerts gegenüber einem in einer Aufzeichnung gespeicherten normalen Blutdruckmesswert, welcher kennzeichnend ist für eine Person ohne eine autonome Fehlfunktion und mit einem ähnlichen Profil wie der Patient,
- Vergleichen des berechneten Herzfrequenzmesswerts gegenüber einem in einer Aufzeichnung gespeicherten normalen Herzfrequenzmesswert, welcher kennzeichnend ist für eine Person ohne autonome Fehlfunktion und mit einem ähnlichen Profil wie der Patient.

6.  System oder Verfahren nach irgendeinem vorhergehenden Anspruch, wobei der normale Blutdruckmesswert die prozentuale Änderung im Blutdruck einer Person ohne autonome Fehlfunktion ist, gemessen während eines autonomen Fehlfunktionstests, bestimmt entsprechend der Formel:

$$NBP = (RBP_{ref} - BBP_{ref})/BBP_{ref},$$

wobei NBP die prozentuale Änderung im Blutdruck der Person ohne autonome Fehlfunktion ist, gemessen während des autonomen Fehlfunktionstests, wobei die Person von einem ähnlichen Profil ist wie der Patient, indem Bezug genommen wird auf die externe Datenbank für den betroffenen Test,
$RBP_{ref}$ die Blutdruckantwort der Person ohne autonome Fehlfunktion ist, wobei die Person von einem ähnlichen Profil ist wie der Patient, was für den ausgeführten autonomen Fehlfunktionstest erwartet wird, und
$BBP_{ref}$ der Ruhe-Blutdruck der Person ohne autonome Fehlfunktion ist, wobei die Person von einem ähnlichen Profil ist wie der Patient, was für den ausgeführten autonomen Fehlfunktionstest erwartet wird.

7.  System oder Verfahren nach Anspruch 6, wobei der tatsächliche Blutdruckmesswert die tatsächliche prozentuale Änderung im Blutdruck des Patienten ist, gemessen während des autonomen Fehlfunktionstests, bestimmt entsprechend der Formel

$$ABP = (RBP_{Patient}\text{-}BBP_{Patient})/BBP_{Patient} \, ,$$

wobei ABP die tatsächliche prozentuale Änderung im Blutdruck des Patienten während des betroffenen autonomen Fehlfunktionstests ist,
$RBP_{Patient}$ die Blutdruckantwort des Patienten auf den ausgeführten autonomen Fehlfunktionstest hin ist und
$BBP_{Patient}$ die Ruhe-Blutdruckantwort des Patienten ist, wie sie für den ausgeführten autonomen Fehlfunktionstest erwartet wird.

8.  System oder Verfahren nach irgendeinem vorhergehenden Anspruch, wobei der normale Herzfrequenzmesswert die prozentuale Änderung in der Herzfrequenz einer Person ohne autonome Fehlfunktion ist, gemessen während eines autonomen Fehlfunktionstests, bestimmt entsprechend der Formel

$$NHR = (RHR_{ref}\text{-}BHR_{ref})/BHR_{ref}$$

wobei NHR die prozentuale Änderung in der Herzfrequenz der Person ohne autonome Fehlfunktion ist, gemessen während des autonomen Fehlfunktionstests, wobei die Person von einem ähnlichen Profil ist wie der Patient, indem Bezug genommen wird auf die externe Datenbank für den betroffenen Test,
$RHR_{ref}$ die Herzfrequenzantwort der Person ohne autonome Fehlfunktion ist, wobei die Person von einem ähnlichen Profil ist wie der Patient, was für den ausgeführten autonomen Fehlfunktionstest erwartet wird, und
$BHR_{ref}$ die Ruhe-Herzfrequenz einer Person ohne autonome Fehlfunktion ist, wobei die Person von einem ähnlichen Profil ist wie der Patient, was für den ausgeführten autonomen Fehlfunktionstest erwartet wird.

9.  System oder Verfahren nach Anspruch 1 oder 8, wobei der tatsächliche Herzfrequenzmesswert die tatsächliche prozentuale Änderung in der Herzfrequenz des Patienten ist, gemessen während des autonomen Fehlfunktionstests, bestimmt entsprechend der Formel

$$AHR = (RHR_{Patient}\text{-}BHR_{Patient})/BHR_{Patient} \, ,$$

wobei AHR die tatsächliche prozentuale Änderung in der Herzfrequenz des Patienten ist, gemessen während des betroffenen autonomen Fehlfunktionstests,
$RHR_{Patient}$ die Herzfrequenzantwort des Patienten auf den ausgeführten autonomen Fehlfunktionstest hin ist und
$BHR_{Patient}$ die Ruhe-Herzfrequenz des Patienten ist, wie sie für den ausgeführten autonomen Fehlfunktionstest erwartet wird.

10. System oder Verfahren nach einem der vorhergehenden Ansprüche, wobei die für jeden autonomen Fehlfunktionstest bestimmten abweichenden Blutdruckwerte vor einem Summieren gewichtet werden und/oder wobei die für jeden autonomen Fehlfunktionstest bestimmten abweichenden Herzfrequenzwerte vor einem Summieren gewichtet werden.

11. System oder Verfahren nach einem der vorhergehenden Ansprüche, wobei der zumindest eine autonome Fehlfunktionstest zumindest einer der folgenden ist:

den Patienten das Valsalva-Manöver ausführen lassen,
den Patienten wiederholt niederlegen und aufstehen lassen,
des Patienten Hand in einem anhaltenden Händedruck halten lassen,
den Patienten einen festen Trainingsplan ausführen lassen und/oder
des Patienten Hand in kaltes Wasser eintauchen lassen.

**EP 2 355 695 B1**

**12.** System oder Verfahren nach einem der vorhergehenden Ansprüche, wobei jeder des zumindest einen autonomen Fehlfunktionstests während einer bestimmten Zeitspanne ausgeführt wird.

**13.** System oder Verfahren nach einem der vorhergehenden Ansprüche, wobei die pulsweise Blutdruck-Überwachungseinrichtung (12) ein nichtinvasiver Blutdruck-Überwachungsdienst ist.

**14.** System nach Anspruch 1, wobei die pulsweise Blutdruck-Überwachungseinrichtung (12) konfiguriert ist, um eine pulsweise Arterien-Pulswelle in Echtzeit zu erhalten.

**15.** System nach Anspruch 1, wobei die Kommunikation von Messungen zwischen der pulsweisen Blutdruck-Überwachungseinrichtung (12) und der Verarbeitungseinheit (18) mittels drahtloser Einrichtungen erfolgt.

**16.** System nach Anspruch 1, wobei die Aufzeichnung der Person, die ein ähnliches Profil aufweist wie der Patient, Informationen in Bezug auf zumindest eines der folgenden Attribute enthält: Alter, Geschlecht, Gesundheitsprofil, Volkszugehörigkeit.

**17.** Mittels eines Computers lesbares Medium, auf dem Software gespeichert ist, die dann, wenn sie durch einen Prozessor eines Systems nach Anspruch 1 ausgeführt wird, ein Verfahren zum Messen von Parametern von autonomen Fehlfunktionstests nach einem der Ansprüche 2 bis 13 ausführt.

**Revendications**

**1.** Système de mesure de paramètres de tests de dysfonction autonome, lequel système comprend :

un dispositif de suivi de pression sanguine battement par battement (12) ; et
une unité de traitement (18),
et est **caractérisé en ce que** le dispositif de suivi de pression sanguine battement par battement est apte à :

obtenir des mesures de pression sanguine battement par battement sur un patient (16) pendant l'exécution de chacun d'au moins un test de dysfonction autonome et calculer des valeurs de pression sanguine variable, de fréquence cardiaque et de fréquence cardiaque variable à partir des mesures de pression sanguine battement par battement pour chacun de l'au moins un test de dysfonction autonome , les valeurs de pression sanguine variable et de fréquence cardiaque variable calculées étant communiquées à l'unité de traitement dans laquelle les valeurs de pression sanguine variable pour chaque test de dysfonction autonome sont sommées et les valeurs de fréquence cardiaque variable calculées pour chaque test de dysfonction autonome sont sommées ; ou bien
le dispositif de suivi de pression sanguine battement par battement (12) est apte à obtenir des mesures de pression sanguine battement par battement sur un patient pendant l'exécution de chacun d'au moins un test de dysfonction autonome et communiquer les mesures à l'unité de traitement (18), l'unité de traitement étant ensuite apte à calculer des valeurs de pression sanguine variable, de fréquence cardiaque et de fréquence cardiaque variable à partir des mesures de pression sanguine battement par battement pour chacun de l'au moins un test de dysfonction autonome, l'unité de traitement (18) étant ensuite apte à sommer les valeurs de pression sanguine variable pour chaque test de dysfonction autonome et sommer les valeurs de fréquence cardiaque variable calculées pour chaque test de dysfonction autonome,
la mesure de pression sanguine variable étant la valeur absolue de la mesure de pression sanguine prise sur le patient moins la mesure de pression sanguine normale stockée dans un enregistrement représentant une personne ayant un profil similaire à celui du patient, la valeur absolue étant multipliée par une constante ;
ou la fréquence cardiaque variable étant la valeur absolue de la mesure de fréquence cardiaque prise sur le patient moins la mesure de fréquence cardiaque normale stockée dans un enregistrement représentant une personne ayant un profil similaire à celui du patient, la valeur absolue étant multipliée par une constante.

**2.** Procédé de mesure de paramètres de tests de dysfonction autonome, lequel procédé comprend :

l'obtention de mesures de pression sanguine battement par battement sur un patient pendant l'exécution de chacun d'au moins un test de dysfonction autonome ;
et est **caractérisé en ce que** le procédé comprend en outre les étapes de :

calcul de valeurs de fréquence cardiaque, de pression sanguine variable et de fréquence cardiaque variable à partir des mesures de pression sanguine battement par battement pour chacun de l'au moins un test de dysfonction autonome ;

sommation des valeurs de pression sanguine variable calculées pour chaque test de dysfonction autonome ; et

sommation des valeurs de fréquence cardiaque variable calculées pour chaque test de dysfonction autonome,

la mesure de pression sanguine variable étant la valeur absolue de la mesure de pression sanguine prise sur le patient moins la mesure de pression sanguine normale stockée dans un enregistrement représentant une personne ayant un profil similaire à celui du patient, la valeur absolue étant multipliée par une constante ; ou la fréquence cardiaque variable étant la valeur absolue de la mesure de fréquence cardiaque prise sur le patient moins la mesure de fréquence cardiaque normale stockée dans un enregistrement représentant une personne ayant un profil similaire à celui du patient, la valeur absolue étant multipliée par une constante.

3. Système ou procédé selon la revendication 1 ou 2, dans lequel l'étape de calcul de valeurs de pression sanguine et de fréquence cardiaque variables à partir des mesures de pression sanguine battement par battement comprend la sous-étape de calcul de la fréquence cardiaque selon la formulé :

$$hr = 60/d,$$

où

hr est la fréquence cardiaque du patient ; et
d est la durée de la forme d'onde de pression sanguine en secondes.

4. Système ou procédé selon la revendication 3, dans lequel la fréquence cardiaque calculée ou la sous-étape de calcul de la fréquence cardiaque est ajustée pour prendre en compte le taux d'échantillonnage du dispositif de suivi de pression sanguine battement par battement (12).

5. Système ou procédé selon l'une quelconque des revendications 1 à 4, dans lequel, pour un système, la mesure de pression sanguine prise sur le patient est comparée à une mesure de pression sanguine normale stockée dans un enregistrement représentant une personne ayant un profil similaire à celui du patient lors de la détermination de la mesure de pression sanguine variable ; la mesure de fréquence cardiaque calculée est comparée à une mesure de fréquence cardiaque normale stockée dans un enregistrement représentant une personne ayant un profil similaire à celui du patient lors de la détermination de la mesure de fréquence cardiaque variable ; et dans lequel, pour un procédé, l'étape de calcul de valeurs de pression sanguine et de fréquence cardiaque variables à partir des mesures de pression sanguine battement par battement comprend les sous-étapes de :

comparaison de la mesure de pression sanguine prise sur le patient à une mesure de pression sanguine normale stockée dans un enregistrement représentant une personne sans dysfonction autonome ayant un profil similaire à celui du patient ;

comparaison de la mesure de fréquence cardiaque calculée à une mesure de fréquence cardiaque normale stockée dans un enregistrement représentant une personne sans dysfonction autonome ayant un profil similaire à celui du patient.

6. Système ou procédé selon l'une quelconque des revendications précédentes, dans lequel la mesure de pression sanguine normale est le pourcentage de changement de la pression sanguine d'une personne sans dysfonction autonome mesurée pendant un test de dysfonction autonome, déterminé selon la formule :

$$NBP = (RBP_{ref} - BBP_{ref})/BBP_{ref}$$

où :

NBP est le pourcentage de changement de la pression sanguine de la personne sans dysfonction autonome pendant le test de dysfonction autonome ; la personne ayant un profil similaire à celui du patient tel que référencé à partir de la base de données externe pour le test concerné ;
$RBP_{ref}$ est la réponse de pression sanguine de la personne sans dysfonction autonome ; la personne ayant un profil similaire à celui du patient tel qu'espéré pour le test de dysfonction autonome qui est effectué ; et
$BBP_{ref}$ est la pression sanguine au repos de la personne sans dysfonction autonome ; la personne ayant un profil similaire à celui du patient tel qu'espéré pour le test de dysfonction autonome qui est effectué.

7. Système ou procédé selon la revendication 6, dans lequel la mesure de pression sanguine réelle est le pourcentage de changement réel de la pression sanguine du patient mesurée pendant le test de dysfonction autonome, déterminé selon la formulé :

$$ABP = (RBP_{patient} - BBP_{patient})/BBP_{patient}$$

où :

ABP est le pourcentage de changement réel de la pression sanguine du patient pendant le test de dysfonction autonome concerné ;
$RBP_{patient}$ est la réponse de pression sanguine du patient pour le test de dysfonction autonome qui est exécuté ; et
$BBP_{patient}$ est la pression sanguine au repos du patient telle qu'espérée pour le test de dysfonction autonome qui est exécuté.

8. Système ou procédé selon l'une quelconque des revendications précédentes, dans lequel la mesure de fréquence cardiaque normale est le pourcentage de changement de la fréquence cardiaque d'une personne sans dysfonction autonome mesurée pendant un test de dysfonction autonome, déterminé selon la formule :

$$NHR = (RHR_{ref} - BHR_{ref})/BHR_{ref}$$

où :

NHR est le pourcentage de changement de la fréquence cardiaque de la personne sans dysfonction autonome mesurée pendant le test de dysfonction autonome ; la personne ayant un profil similaire à celui du patient tel que référencé à partir de la base de données externe pour le test concerné ;
$RHR_{ref}$ est la réponse de fréquence cardiaque de la personne sans dysfonction autonome ; la personne ayant un profil similaire à celui du patient tel qu'espéré pour le test de dysfonction autonome qui est effectué ; et
$BHR_{ref}$ est la fréquence cardiaque au repos de la personne sans dysfonction autonome ; la personne ayant un profil similaire à celui du patient tel qu'espéré pour le test de dysfonction autonome qui est effectué.

9. Système ou procédé selon la revendication 1 ou la revendication 8, dans lequel la mesure de fréquence cardiaque réelle est le pourcentage de changement réel de la fréquence cardiaque du patient mesurée pendant le test de dysfonction autonome, déterminé selon la formule :

$$AHR = (RHR_{patient} - BHR_{patient})/BHR_{patient}$$

où :

AHR est le pourcentage de changement réel de la fréquence cardiaque du patient mesurée pendant le test de dysfonction autonome concerné ;
$RHR_{patient}$ est la réponse de fréquence cardiaque du patient pour le test de dysfonction autonome qui est exécuté ; et
$BHR_{patient}$ est la fréquence cardiaque au repos du patient telle qu'espérée pour le test de dysfonction autonome qui est exécuté.

10. Système ou procédé selon l'une quelconque des revendications précédentes, dans lequel les valeurs de pression sanguine variable déterminées pour chaque test de dysfonction autonome sont pondérées avant sommation ; et/ou les valeurs de fréquence cardiaque variable déterminées pour chaque test de dysfonction autonome sont pondérées avant sommation.

**11.** Système ou procédé selon l'une quelconque des revendications précédentes, dans lequel l'au moins un test de dysfonction autonome est au moins l'un de ce qui suit :

le fait que le patient effectue une manoeuvre de Valsalva ;
le fait que le patient se couche et se lève de manière répétitive ;
le fait que le patient serre sa main d'une manière soutenue sur une poignée ;
le fait que le patient suive un régime d'exercices défini ; et/ou
le fait que le patient immerge sa main dans l'eau froide.

**12.** Système ou procédé selon l'une quelconque des revendications précédentes, dans lequel chacun de l'au moins un test de dysfonction autonome est exécuté pendant un laps de temps prédéterminé.

**13.** Système ou procédé selon l'une quelconque des revendications précédentes, dans lequel le dispositif de suivi de pression sanguine battement par battement (12) est un service de suivi de pression sanguine non invasif.

**14.** Système selon la revendication 1, dans lequel le dispositif de suivi de pression sanguine battement par battement (12) est configuré pour obtenir une onde de pouls artériel battement par battement en temps réel.

**15.** Système selon la revendication 1, dans lequel une communication de mesures entre le dispositif de suivi de pression sanguine battement par battement (12) et l'unité de traitement (18) se fait par des moyens sans fil.

**16.** Système selon la revendication 1, dans lequel l'enregistrement de la personne ayant un profil similaire à celui du patient inclut des informations concernant au moins l'un des attributs suivants : âge ; sexe ; profil de santé ; ethnicité.

**17.** Support lisible par un ordinateur sur lequel est stocké un logiciel qui, lorsqu'il est exécuté par un processeur d'un système selon la revendication 1, exécute un procédé de mesure de paramètres de tests de dysfonction autonome selon l'une quelconque des revendications 2 à 13.

Calibrate non-invasive blood pressure
monitoring device and obtain resting
beat-to-beat blood pressure of patient — 110

Commence/Repeat Autonomic
dysfunction test on patient to obtain
beat-to-beat blood pressure
measurements — 120/135

180

Store beat-to-beat blood
pressure measurements in
database — 130

Display results

140

Does blood pressure
measurement satisfy
set of criteria?

NO

YES

Perform quantitative
assessment to determine
the extent of autonomic
dysfunction of the
patient — 170

145

All autonomic
dysfunction tests
completed?

NO

YES

Compare variant heart
rate and blood pressures
values with that of a
normal person with a
similar profile in
database — 160

Calculate beat-to-beat heart rate
based on the beat-to-beat blood
pressure obtained — 150

**FIGURE 1**

FIGURE 2

**FIGURE 3**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2006050725 A1

- US 6416473 B1